# EUROPEAN PATENT SPECIFICATION

(11) **EP 4 559 899 B1**
(45) Date of publication and mention of the grant of the patent: **01.04.2026**
(21) Application number: 24213940.0
(22) Date of filing: 19.11.2024
(51) Int. Cl.: C07D 209/08, A61P 9/12, A61P 9/04, A61K 31/404

(54) **SYNTHESIS OF 1-AMINO-2-METHYL-2,3-DIHYDROINDOLE BY NITROSATING 2-METHYL-2,3-DIHYDRO-1H-INDOLE AND THEN REDUCING THE OBTAINED 1-NITROSO-2-METHYL-2,3-DIHYDROINDOLE WITH THIOUREA DIOXIDE**
SYNTHESE VON 1-AMINO-2-METHYL-2,3-DIHYDROINDOL DURCH NITROSIERUNG VON 2-METHYL-2,3-DIHYDRO-1H-INDIDOL UND ANSCHLIESSENDE REDUKTION DES ERHALTENEN 1-NITROSO-2-METHYL-2,3-DIHYDROINDOLS MIT THIOHARNSTOFFDIOXID
SYNTHÈSE DU 1-AMINO-2-MÉTHYL-2,3-DIHYDROINDOLE PAR NITROSATION DU 2-MÉTHYL-2,3-DIHYDRO-1H-INDOLE PUIS RÉDUCTION DU 1-NITROSO-2-MÉTHYL-2,3-DIHYDROINDOLE OBTENU AVEC DU DIOXYDE DE THIOURÉE

(30) Priority: 21.11.2023 EP 23211166
(43) Date of publication of application: 28.05.2025
(73) Proprietor: KRKA, d.d., Novo mesto, 8000 Novo mesto (SI)
(72) Inventor: Gorenc, Ana, 8000 Novo mesto (SI); Kralj, David, 8000 Novo mesto (SI)
(74) Representative: Kutzenberger Wolff & Partner

(56) References cited:
- CN-A- 103 467 355
- CHAUDHARY PRIYANKA ET AL: "Green Chemistry COMMUNICATION Cite this: Green Chem A metal free reduction of aryl-N-nitrosamines to the corresponding hydrazines using a sustainable reductant thiourea dioxide View Article Online View Journal | View Issue", GREEN CHEM, vol. 18, 3 October 2016 (2016-10-03), pages 6215 - 6221, XP055844385, DOI: 10.1039/c6gc02444k
- FRANCISZEK S - ET AL: "1-[(Imidazolin-2-yl)amino]indoline and 1-[(imidazolin-2-yl)amino]1,2,3,4-tetrahydroquinoline derivatives: new insights into their circulatory activities", ACTA POLONIA PHAMACEUTICA - DRUG RESEARCH, vol. 72, no. 2, 2015, PL, pages 277 - 287, XP093259084, ISSN: 0001-6837

## Description

Priority is claimed of European patent application no. 23 211 166.6 that was filed on November 21, 2023.

The invention relates to a process for the synthesis of 1-amino-2-methyl-2,3-dihydroindole which is a key intermediate in the industrial synthesis of indapamide. The process involves nitrosation of 2-methyl-2,3-dihydroindole and subsequent reduction of the N-nitroso group with thiourea dioxide (TDO) as reducing agent.

Indapamide (ATC C03BA11) has antihypertensive properties and is used in the treatment of essential arterial hypertension. Indapamide (N-(3-sulfamyl-4-chloro-benzamido)-2-methyl-2,3-dihy-droindole, CAS 26807-65-8) has the following chemical structure:

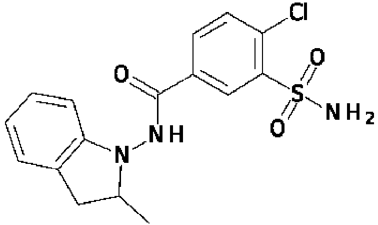

Several methods for the synthesis of indapamide and its intermediates are already known.

US 4,570,001 discloses cyclizing 1-allyl-1-phenyl-2-(3-sulphamoyl-4-chlorobenzoyl)hydrazine in the presence of Lewis or Bronsted acids:

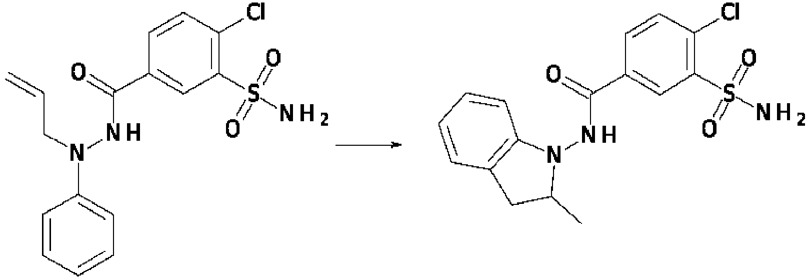

However, such cyclisation results in the formation of a large number of secondary products and the yield of indapamide is hardly satisfactory.

US 3,565,911 discloses the reaction of 4-chloro-3-sulphamoylbenzoyl chloride with 1-amino-2-methyl-2,3-dihydroindole:

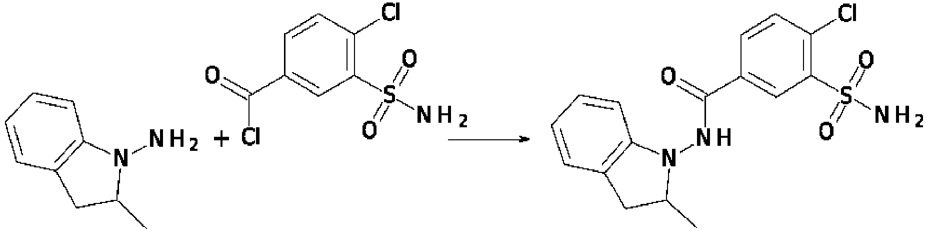

The key intermediate 1-amino-2-methyl-2,3-dihydroindole can be prepared by reducing the N-nitroso analogue 1-nitroso-2-methyl-2,3-dihydroindole in accordance with J.B. Wright and R.E. Wil-lette, J. Med. and Pharm. Chem. 5, 1962, 811:

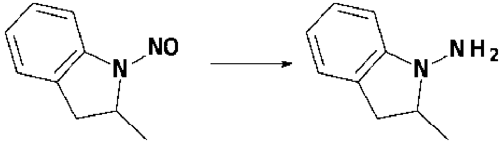

However, the yield of the various stages of that process is hardly satisfactory. In addition, the process results in the production of secondary products having potential toxicity.

According to the review J.P. Anselme, The Organic Chemistry ofN-Nitrosamines: A Brief Review, ACS Symposium Series, 1979, 1-12, the most common method to perform the transformation of N-nitrosamines to 1,1-disubstituted hydrazines has been zinc dust in acid, generally acetic acid; tetrazenes are sometimes formed as by-products and denitrosation can also occur. Several other reducing methods have been investigated; reduction with lithium aluminum hydride (LiAlH₄) and catalytic hydrogenation are sometimes useful. Sodium dithionite (Na₂S₂O₄) reduction of benzyl substituted N-nitrosamines in base can result in fragmentation to the hydrocarbons (Overberger-Lombardino reaction).

US 5,101,040 discloses the synthesis of the key intermediate 1-amino-2-methyl-2,3-dihydroindole by reacting 2-methyl-2,3-dihydro-1H-indole with monochloramine at a yield of 85%:

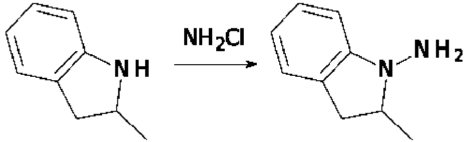

The preparation of chloramine is relatively cheap, as it is produced by the reaction of ammonia with sodium hypochlorite. However, the toxicity of chloramine and its instability in aqueous solutions at room temperature are among the reasons why it is not often used in syntheses in batch reactors.

US 5,110,946 discloses the synthesis of the key intermediate 1-amino-2-methyl-2,3-dihydroindole by reacting 2-methyl-2,3-dihydro-1H-indole with hydroxylamine-O-sulphonic acid at a yield of 78% (see also P. Wang et al., Transactions of Beijing institute of Technology, 2001, (4): 531-534):

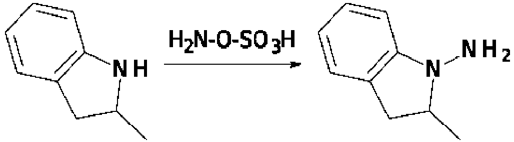

According to the information provided in US 5,110,946, Japanese patent 54-030159 describes a process for the preparation of indapamide from 4-chloro-3-sulphamoyl-N-(2-methylindol-1-yl)benzamide which is converted into indapamide after reduction. That process also leads to the formation of a large number of secondary products and several purifications are necessary to obtain a pharmaceutical quality product.

Further, with respect to indapamide and imidazoline-containing agents, reference can be made to CN 103 467 355 A and Saczewski et al., ACTA POLONIAE PHARMACEUTICA - DRUG RESEARCH, vol. 72, no. 2, 2015, pp. 277-287 (XP093259084A).

The known processes for the synthesis of indapamide do not provide indapamide at a satisfactory degree of purity and/or yield. Furthermore, certain stages of those processes pose problems from an industrial standpoint. In view of the therapeutic value of indapamide and the importance of an industrial process enabling it to be obtained with a satisfactory degree of purity, a good yield and, if possible, from fairly inexpensive commercially available starting materials, there is a demand for improved processes.

It is an object of the invention to provide an improved process for the synthesis of indapamide and its key intermediate 1-amino-2-methyl-2,3-dihydroindole. The process should provide satisfactory yields and high purities at minimized byproduct formation. It should allow for synthesis on industrial scale. It should be inexpensive, employ starting materials that are easy to obtain, and avoid harmful reagents.

This object has been achieved by the subject-matter of the patent claims.

It has been surprisingly found that 1-nitroso-2-methyl-2,3-dihydroindole can be reduced with thiourea dioxide under milder conditions with greater selectivity and yield of the desired product 1-amino-2-methyl-2,3-dihydroindole.

Thiourea dioxide (TDO, TDU, TUD, also known as s formamidinesulfinic acid (FASA) or aminoimino methane sulfinic acid (AIMS), Thiox) is an organosulfur compound that is known as a *"green"* industrial reducing agent. It is predominantly used in the textile industry. S.V. Makarov et al. Chem. Eur. J. 2014, 20, 14164-14176 provides an overview on developments in the chemistry and applications of thiourea mono-, di-, and trioxide including organocatalysis; phase-transfer and polymerization reactions; reduction of graphene and graphite oxides; bitumen modifications; synthesis of guanidines and their derivatives; and the study of nonlinear dynamical phenomena in chemical kinetics. S.V. Makarov et al., J. Phys. Chem. B 2001, 105, 12634-12643 reports about decomposition kinetics of a series of thiourea dioxides that has been studied in alkaline media. Thiourea dioxide has been suggested as organocatalyst embedded in PEG, for oxidation of sulfides to sulfoxides, for regio- and stereoselective ring-opening of epoxides, for the synthesis of benzimidazoles, and for the preparation of N-formamidi-nylamino acids (R. Ayazi-Nasrabadi, Synlett 2015, 26, 1281-1282). P. Chaudhary et al., Green Chem. 2016, 18, 6215 relates to the metal free reduction of aryl-N-nitrosamines to the corresponding hydrazines using a sustainable reductant thiourea dioxide. However, yields hardly exceed 80%.

Further, it has been surprisingly found that 2-methyl-2,3-dihydroindole can be nitrosated and subsequently reduced with thiourea dioxide as reducing agent under basic conditions in a one-pot process, i.e. without isolation of the nitroso intermediate, i.e. 1-nitroso-2-methyl-2,3-dihydroindole. The reaction is highly selective and can be carried out in aqueous alcoholic medium, e.g. aqueous methanol.

Alternative reducing agents such as zinc, lithium aluminum hydride (LiAlH₄) or sodium dithionite (Na₂S₂O₄) have significantly lower selectivity than thiourea dioxide. Further, reduction by means of thiourea dioxide is not only highly selective but additionally leads to non-toxic by-products (urea and sodium salts). Still further, thiourea dioxide is easy to handle for batch reactor application. By using thiourea dioxide, metal waste after the reaction can be avoided which is otherwise obtained when using titanium reagent (e.g. TiCl₄/Mg) and zinc-acetic acid (Zn/AcOH). Thiourea dioxide is more selective than other organosulfur compounds and the conversion is achieved under milder conditions than e.g. described by Guo, Wei; et al: Photocatalyzed intermolecular amination for the synthesis of hydrazonamides. Org. Chem. Front. 2021, 8(14), 3838-3846.

The isolation of 1-amino-2-methyl-2,3-dihydroindole as its hydrochloride salt after the reaction is also simplified in comparison to reductions by means of other reducing agents, because the by-products after the reduction with thiourea dioxide are dissolved in water and the desired 1-amino-2-methyl-2,3-dihydroindole can simply be extracted with an organic solvent.

A first aspect of the invention relates to a process for the synthesis of key intermediate 1-amino-2-methyl-2,3-dihydroindole **3** (also referred to as 1-amino-2-methyl-indoline). The starting material 2-methyl-2,3-dihydro-1H-indole **1** (also referred to as 2-methylindoline) is selectively nitrosated at its secondary amino group to afford 1-nitroso-2-methyl-2,3-dihydroindole **2** (also referred to as 1-nitroso-2-methyl-indoline); which in turn is subsequently reduced with thiourea dioxide (TDO) to afford 3:

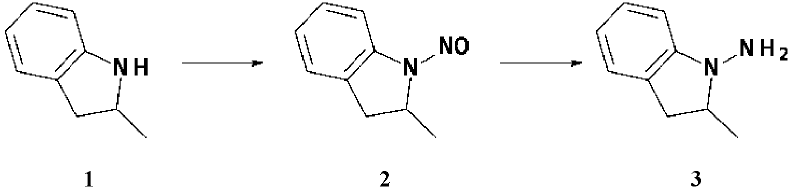

The thus obtained 1-amino-2-methyl-2,3-dihydroindole 3 can be stabilized by conversion into its hydrochloride salt 4:

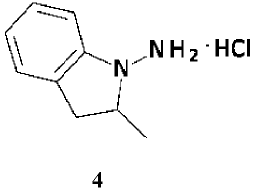

Both, *"nitrosylation"* and *"nitrosation"* reactions formally result in the addition of an *"NO"* moiety onto another molecule, here the secondary amino group of 2-methyl-2,3-dihydro-1H-indole **1.** As a consequence, for the purpose of the specification, *"nitrosylation"* and *"nitrosation"* can be used interchangeably. Strictly speaking, however, *"nitrosylation"* is a radical reaction involving NO, whereas *"nitrosation"* is the formal addition of a NO⁺ cation. Both are contemplated according to the invention.

Nitrosation of 2-methyl-2,3-dihydro-1H-indole **1** may e.g. be carried out with sodium nitrite (NaNO₂), which in the presence of acid forms a nitrosyl cation (NO⁺), which then reacts with a secondary amine to form the N-nitroso compound 1-nitroso-2-methyl-2,3-dihydroindole **2.**

In the process of the invention, 1-amino-2-methyl-2,3-dihydroindole **3** may be obtained through the nitrosation of 2-methyl-2,3-dihydro-1H-indole **1,** wherein the resulting nitroso intermediate **2** is subsequently reduced to the hydrazine 1-amino-2-methyl-2,3-dihydroindole **3** in the last step.

The process according to the invention is for the synthesis of 1-amino-2-methyl-2,3-dihydroindole **3** or an acid addition salt thereof, preferably 1-amino-2-methyl-2,3-dihydroindole hydrochloride **4,** and comprises the steps of
(a) providing 2-methyl-2,3-dihydro-1H-indole **1** 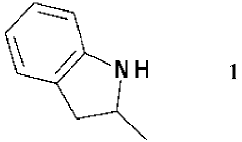
(b) nitrosating the 2-methyl-2,3-dihydro-1H-indole **1** with a nitrosation agent to afford 1-nitroso-2-methyl-2,3-dihydroindole **2** 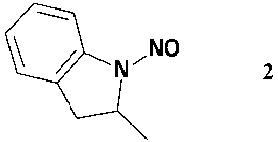
(c) reducing the 1-nitroso-2-methyl-2,3-dihydroindole 2 with thiourea dioxide as reducing agent to afford the 1-amino-2-methyl-2,3-dihydroindole 3 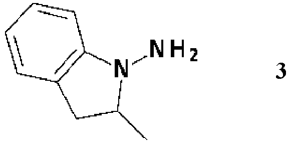 and
(d) optionally, converting the 1-amino-2-methyl-2,3-dihydroindole **3** into the acid addition salt thereof.

In step (a) of the process according to the invention, 2-methyl-2,3-dihydro-1H-indole 1 is provided. Methods to provide 2-methyl-2,3-dihydro-1H-indole 1 are known from the prior art and for the purposes of the invention are not particularly limited. 2-methyl-2,3-dihydro-1H-indole 1 is also commercially available.

In step (b) of the process according to the invention, the 2-methyl-2,3-dihydro-1H-indole **1** is nitrosated with a nitrosation agent to afford 1-nitroso-2-methyl-2,3-dihydroindole **2.**

Preferably, the nitrosating agent in step (b) is a nitrite; preferably an alkaline metal nitrite; more preferably sodium nitrite (NaNO₂).

Preferably, step (b) is performed in the presence of an acid; preferably an organic acid; more preferably a carboxylic acid; still more preferably a monocarboxylic acid; yet more preferably an aliphatic monocarboxylic acid; even more preferably acetic acid.

Preferably, step (b) is performed in an aqueous solvent. Preferably, the aqueous solvent contains an alcohol; preferably selected from methanol (MeOH), ethanol (EtOH) and isopropanol (i-PrOH); more preferably methanol (MeOH).

Preferably, step (b) is performed at a temperature within the range of from -5 to 45°C; preferably 0 to 40°C; more preferably 5 to 35°C; still more preferably 10 to 30°C; and yet more preferably 15 to 25°C.

Preferably, step (b) is performed for a duration within the range of from 1 to 10 hours; more preferably 2 to 9 hours; still more preferably 3 to 8 hours; and yet more preferably 4 to **7** hours.

In step (c) of the process according to the invention, the 1-nitroso-2-methyl-2,3-dihydroindole **2** is reduced with thiourea dioxide (TDO) as reducing agent to afford the 1-amino-2-methyl-2,3-dihy-droindole **3.**

Preferably, in step (c) the molar ratio of the thiourea dioxide to the 1-nitroso-2-methyl-2,3-di-hydroindole **2** is at most 8; preferably at most 7; more preferably at most 6; still more preferably at most 5; yet more preferably at most 4; even more preferably at most 3; most preferably at most 2.5; and in particular at most 2.

Preferably, step (c) is performed in an aqueous solvent. Preferably, the aqueous solvent contains an alcohol; preferably selected from methanol, ethanol and isopropanol; more preferably methanol. Preferably, the volume ratio of the alcohol to water is within the range of from 1:5 to 5:1.

Preferably, step (c) is performed in the presence of a base; preferably an inorganic base; more preferably a hydroxide; still more preferably an alkaline metal hydroxide; yet more preferably sodium hydroxide.

Preferably, step (c) is performed at a temperature within the range of from 40 to 75°C; preferably 45 to 70°C; more preferably 50 to 70°C; still more preferably 55 to 65°C.

Preferably, step (c) is performed for a duration of 0.5 to 72 hours; more preferably 1 to 24 hours.

In preferred embodiments of the process according to the invention, steps (b) and (c) are performed in the same reactor without isolating the 1-nitroso-2-methyl-2,3-dihydroindole **2.**

In optional step (d) of the process according to the invention, the 1-amino-2-methyl-2,3-dihy-droindole **3** is converted into an acid addition salt thereof.

Preferably, the acid addition salt in step (d) is the hydrochloride.

Preferably, step (d) involves contacting the 1-amino-2-methyl-2,3-dihydroindole **3** with HCl in a solvent; preferably in ethyl acetate (EA), ethanol (EtOH), isopropanol (i-PrOH), cyclopentyl methyl ether (CPME) or water.

In preferred embodiments, a solution of 1-amino-2-methyl-2,3-dihydroindole **3,** preferably in toluene or ethyl acetate, is contacted with a solution of HCl, preferably with an aqueous solution of HCl.

In other preferred embodiments, a solution of 1-amino-2-methyl-2,3-dihydroindole **3,** preferably in toluene or ethyl acetate, is contacted with a solution of HCl in a solvent selected from ethyl acetate, ethanol (EtOH), isopropanol (i-PrOH), cyclopentyl methyl ether (CPME), aqueous ethyl acetate, aqueous ethanol (EtOH aq.), aqueous isopropanol (i-PrOH aq.), and aqueous cyclopentyl methyl ether (CPME aq.).

Another aspect of the invention relates to a process for the synthesis of indapamide comprising the process for the synthesis of 1-amino-2-methyl-2,3-dihydroindole **3** or an acid addition salt thereof according to the invention as described above.

Preferably, the process comprises the steps of
(e) providing 4-chloro-3-sulphamoylbenzoyl chloride **5** 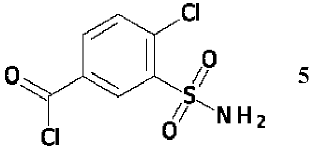 and
(f) reacting the 4-chloro-3-sulphamoylbenzoyl chloride **5** with the 1-amino-2-methyl-2,3-dihydroindole **3** or the acid addition salt thereof to afford indapamide.

In step (e) of the process according to the invention, 4-chloro-3-sulphamoylbenzoyl chloride **5** is provided. Methods to provide 4-chloro-3-sulphamoylbenzoyl chloride **5** are known from the prior art (see e.g. US 3,565,911, US 5,101,040, US 5,110,946) and for the purposes of the invention are not particularly limited.

In step (e) of the process according to the invention, the 4-chloro-3-sulphamoylbenzoyl chloride **5** is reacted with the 1-amino-2-methyl-2,3-dihydroindole **3** or the acid addition salt thereof to afford indapamide. Suitable reaction conditions are known from the prior art (see e.g. US 3,565,911, US 5,101,040, US 5,110,946, CN 101 717 359 B) and for the purposes of the invention are not particularly limited.

A skilled person immediately recognizes that as an alternative to the benzoyl chloride, other common activation methods for coupling carboxylic acids with amines may be used (see e.g. B. Ziobro et al., Journal of Heterocyclic Chemistry, 200.

The following examples further illustrate the invention:

### Example 0 - nitrosation of 1 to afford 2:

Sodium nitrite (1.04 kg) was suspended in water (2 L) and methanol (6.8 L). 2-methyl-2,3-dihydro-1H-indole **1** was added (1 kg) to the mixture followed by the addition of acetic acid (0.64 L) at 17-23°C. After completion of the addition, the reaction mixture was stirred at 15-25°C for 4-7 h.

### Comparative examples 1-1 to 1-5 - reduction of 2 with zinc:

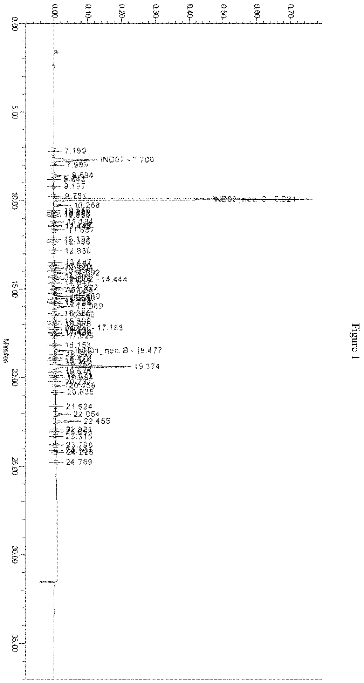

The 1-nitroso-2-methyl-2,3-dihydroindole **2** obtained in Example 0 was reduced with zinc under slightly acidic conditions using NH₄Cl in MeOH/H₂O. Various equivalent amounts of zinc were tested ranging from 3.6 to 10 eq. The reduction was non-selective (see Figure 1) and yielded significant amounts of the starting material 2-methyl-2,3-dihydro-1H-indole **1.**

This deteriorates the quality of the desired product 1-amino-2-methyl-2,3-dihydroindole hydrochloride **4,** since 2-methyl-2,3-dihydro-1H-indole **1** likewise forms a hydrochloride.

Figure 1 shows a representative chromatogram (HPLC-purity) of the reaction mixture.

### Comparative example 2 - reduction of 2 with LiAlH₄:

When using LiAlH₄ for reducing 1-nitroso-2-methyl-2,3-dihydroindole **2** obtained in Example 0, the reaction mixture turned dark purple. The content of 1-amino-2-methyl-2,3-dihydroindole **3** in the isolated product was 90.7%, but due to the lower efficiency and sensitivity of the reducing reagent, no further development of synthesis was pursued. When handling LiAlH₄, care must be taken to avoid contact with moisture (inert atmosphere, anhydrous solvents) and because the reaction releases hydrogen.

### Comparative example 3 - reduction of 2 with Na₂S₂O₄:

The reduction of 1-nitroso-2-methyl-2,3-dihydroindole **2** obtained in Example 0 to 1-amino-2-methyl-2,3-dihydroindole **3** with Na₂S₂O₄ was performed under relatively harsh conditions (7 eq., Tᵣ = 92±2°C). Given the poor efficiency and lower quality of isolated 1-amino-2-methyl-2,3-dihydroindole hydrochloride **4** (HPLC: yield of **3:** 89.08%; residual **1:** 10.36%), no further development of the synthesis was carried out.

### Inventive examples 4-1 to 4-16 - reduction of 2 with thiourea dioxide (TDO):

A solution of NaOH (3 kg) in water (5.34 L) was prepared in a separate reactor and stirred until dissolved. The aqueous solution of sodium hydroxide thus prepared was added to the reaction mixture obtained in Example 0 in the temperature range of 20-40°C. After the total amount of base had been added, the reaction mixture was cooled to 20-30°C. Thiourea dioxide (1.93 kg) was charged into the reactor. After the addition, the reaction mixture was gradually heated to 50-70°C. When the final temperature was reached, the reaction mixture was continually stirred at this temperature to the appropriate conversion, and the reaction mixture was sampled and analyzed by TLC.

### Work-up and formation of hydrochloride salt:

After completion of the reaction, water (20 L) was poured into the suspension. The mixture was heated to 55±5°C and stirred. Toluene (10 L) was charged into the reactor and the mixture was again heated to 55±5°C. When stirring was finished, the phases separated and toluene (10 L) was added to the aqueous phase.

The organic phases were separated and 37% HCl solution (0.941 L) was slowly added to the organic phase in the temperature range of 17-23°C. After the entire addition, the crystallization mixture was stirred for 3±1 h at 20±3°C.

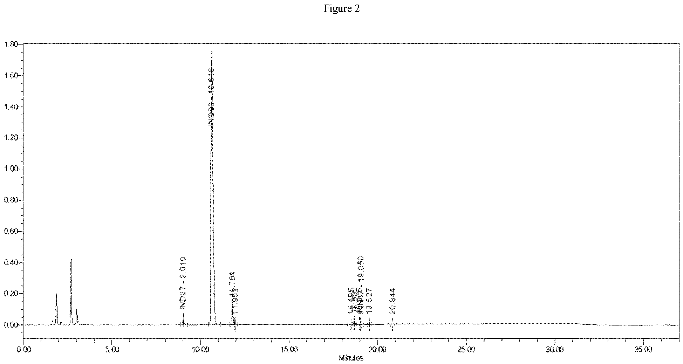

The product was isolated and washed with ethyl acetate (1.67 L). The isolated 1-amino-2-methyl-2,3-dihydroindole hydrochloride **4** was sufficiently pure to be used for the synthesis of indapamide without any additional purification. Figure 2 shows a representative chromatogram (HPLC-purity) of the reaction mixture.

The table below lists the various attempts of reducing 1-nitroso-2-methyl-2,3-dihydroindole **2.** HPLC-purity values are determined on isolated 1-amino-2-methyl-2,3-dihydroindole hydrochloride **4:**

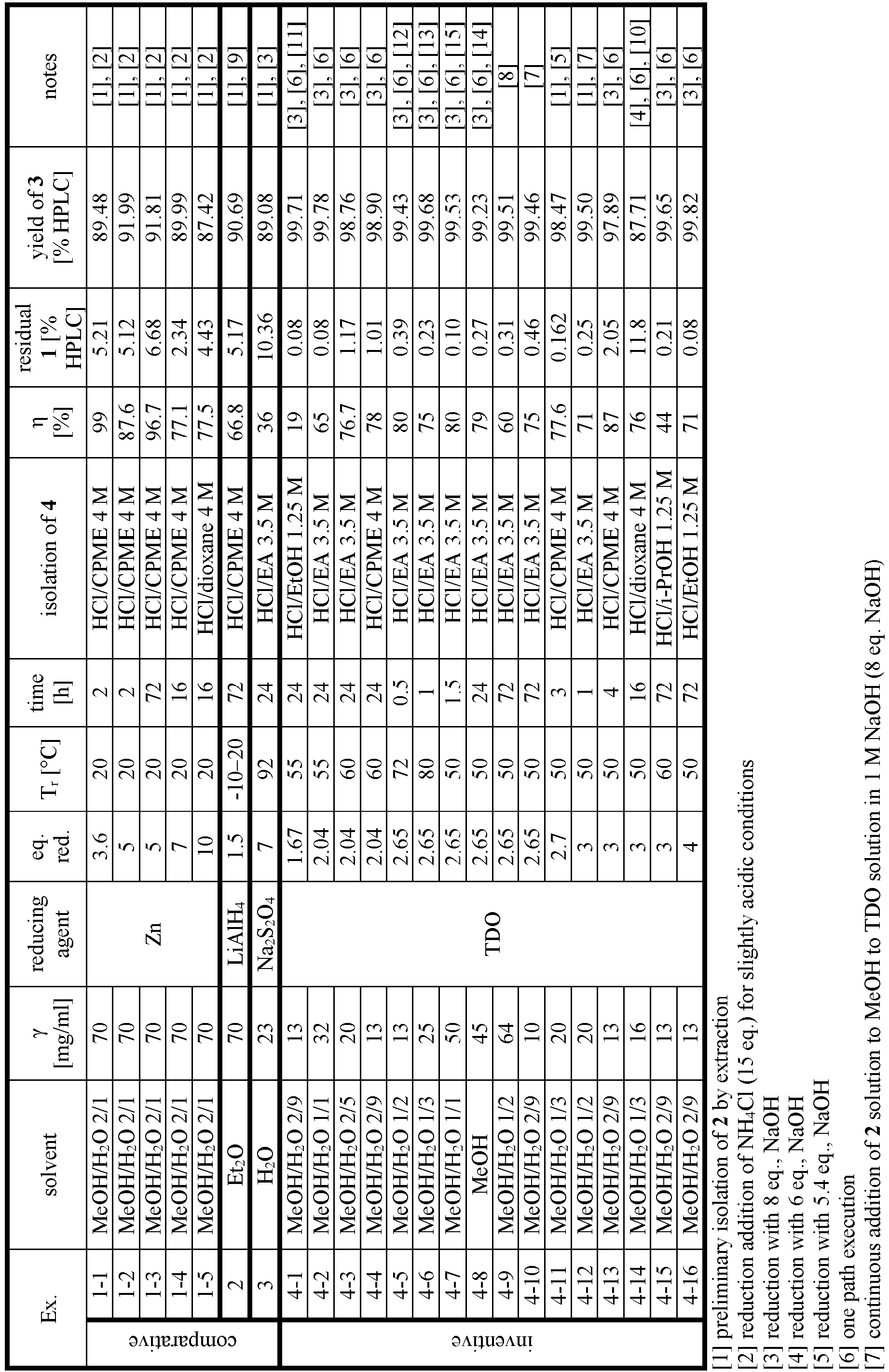

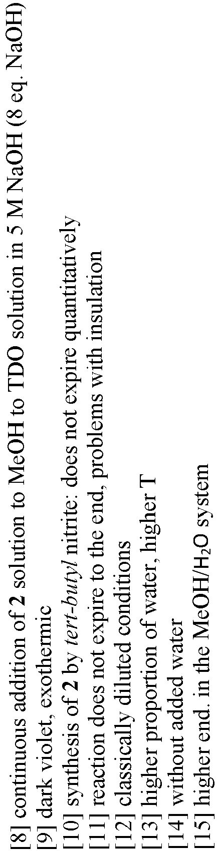

## Claims

1. A process for the synthesis of 1-amino-2-methyl-2,3-dihydroindole **3** or an acid addition salt thereof comprising the steps:
(a) providing 2-methyl-2,3-dihydro-1H-indole **1** 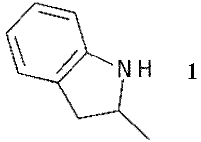
(b) nitrosating the 2-methyl-2,3-dihydro-1H-indole **1** with a nitrosation agent to afford 1-nitroso-2-methyl-2,3-dihydroindole **2** 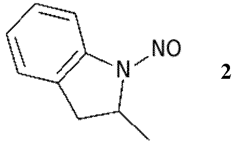
(c) reducing the 1-nitroso-2-methyl-2,3-dihydroindole 2 with thiourea dioxide as reducing agent to afford the 1-amino-2-methyl-2,3-dihydroindole 3 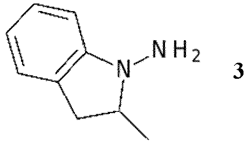 and
(d) optionally, converting the 1-amino-2-methyl-2,3-dihydroindole 3 into the acid addition salt thereof.

2. The process according to claim 1, wherein in step (c) the molar ratio of the thiourea dioxide to the 1-nitroso-2-methyl-2,3-dihydroindole **2** is at most 8; preferably at most 7; more preferably at most 6; still more preferably at most 5; yet more preferably at most 4; even more preferably at most 3; most preferably at most 2.5; and in particular at most 2.

3. The process according to claim 1 or 2, wherein step (c) is performed in an aqueous solvent.

4. The process according to claim 3, wherein the aqueous solvent contains an alcohol; preferably selected from methanol, ethanol and isopropanol; more preferably methanol.

5. The process according to claim 4, wherein the volume ratio of the alcohol to water is within the range of from 1:5 to 5:1.

6. The process according to any of the preceding claims, wherein step (c) is performed at a temperature within the range of from 40 to 75, preferably 45 to 70, more preferably 50 to 70, most preferably 55 to 65.

7. The process according to any of the preceding claims, wherein step (c) is performed in the presence of a base; preferably an inorganic base; more preferably a hydroxide; still more preferably an alkaline metal hydroxide; yet more preferably sodium hydroxide.

8. The process according to any of the preceding claims, wherein the nitrosating agent in step (b) is a nitrite; preferably an alkaline metal nitrite; more preferably sodium nitrite.

9. The process according to any of the preceding claims, wherein step (b) is performed in an aqueous solvent.

10. The process according to claim 9, wherein the aqueous solvent contains an alcohol; preferably selected from methanol, ethanol and isopropanol; more preferably methanol.

11. The process according to any of the preceding claims, wherein steps (b) and (c) are performed in the same reactor without isolating the 1-nitroso-2-methyl-2,3-dihydroindole **2.**

12. The process according to any of the preceding claims, wherein the acid addition salt in step (d) is the hydrochloride.

13. The process according to any of the preceding claims, wherein step (d) involves contacting the 1-amino-2-methyl-2,3-dihydroindole **3** with HCl in a solvent; preferably in ethyl acetate, ethanol, isopropanol, cyclopentyl methyl ether, water, aqueous ethyl acetate, aqueous ethanol, aqueous isopropanol, or aqueous cyclopentyl methyl ether.

14. A process for the synthesis of indapamide comprising the process for the synthesis of 1-amino-2-methyl-2,3-dihydroindole **3** or an acid addition salt thereof according to any of the preceding claims.

15. The process according to claim 14, which comprises the steps of
(e) providing 4-chloro-3-sulphamoylbenzoyl chloride **5** 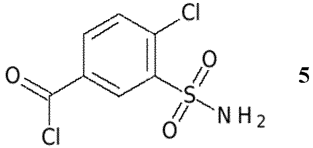 and
(f) reacting the 4-chloro-3-sulphamoylbenzoyl chloride **5** with the 1-amino-2-methyl-2,3-dihydroindole **3** or the acid addition salt thereof to afford indapamide.

## Patentansprüche

1. Ein Verfahren zur Synthese von 1-Amino-2-methyl-2,3-dihydroindol **3** oder eines Säureadditionssalzes davon, umfassend die Schritte:
(a) Bereitstellung von 2-Methyl-2,3-dihydro-1H-indol 1 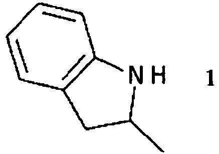
(b) Nitrosierung des 2-Methyl-2,3-dihydro-1H-indols 1 mit einem Nitrosierungsmittel, um 1-nitroso-2-methyl-2,3-dihydroindol 2 zu erhalten 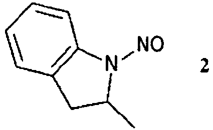
(c) Reduktion des 1-Nitroso-2-methyl-2,3-dihydroindols 2 mit Thioharnstoffdioxid als Reduktionsmittel zur Bildung des 1-Amino-2-methyl-2,3-dihydroindols 3 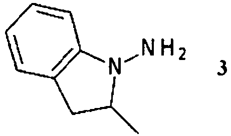 und
(d) gegebenenfalls die Umwandlung des 1-Amino-2-methyl-2,3-dihydroindols 3 in dessen Säureadditionssalz.

2. Das Verfahren nach Anspruch 1, wobei in Schritt (c) das molare Verhältnis des Thioharnstoffdioxids zu dem 1-Nitroso-2-methyl-2,3-dihydroindol **2** höchstens 8, vorzugsweise höchstens 7, noch bevorzugter höchstens 6, noch bevorzugter höchstens 5, noch bevorzugter höchstens 4, noch bevorzugter höchstens 3, am meisten bevorzugt höchstens 2,5 und insbesondere höchstens 2 beträgt.

3. Das Verfahren nach Anspruch 1 oder 2, wobei Schritt (c) in einem wässrigen Lösungsmittel durchgeführt wird.

4. Das Verfahren nach Anspruch 3, wobei das wässrige Lösungsmittel einen Alkohol enthält, vorzugsweise ausgewählt aus Methanol, Ethanol und Isopropanol, besonders bevorzugt Methanol.

5. Das Verfahren nach Anspruch **4,** wobei das Volumenverhältnis von Alkohol zu Wasser im Bereich von 1:5 bis 5:1 liegt.

6. Das Verfahren nach einem der vorhergehenden Ansprüche, wobei Schritt (c) bei einer Temperatur im Bereich von 40 bis 75, vorzugsweise 45 bis 70, besonders bevorzugt 50 bis 70, am meisten bevorzugt 55 bis 65 durchgeführt wird.

7. Das Verfahren nach einem der vorangehenden Ansprüche, wobei Schritt (c) in Gegenwart einer Base durchgeführt wird, vorzugsweise einer anorganischen Base, noch bevorzugter eines Hydroxids, noch bevorzugter eines Alkalimetallhydroxids, noch bevorzugter Natriumhydroxid.

8. Das Verfahren nach einem der vorhergehenden Ansprüche, wobei das Nitrosierungsmittel in Schritt (b) ein Nitrit ist, vorzugsweise ein Alkalimetallnitrit, besonders bevorzugt Natriumnitrit.

9. Das Verfahren nach einem der vorangehenden Ansprüche, wobei Schritt (b) in einem wässrigen Lösungsmittel durchgeführt wird.

10. Das Verfahren nach Anspruch 9, wobei das wässrige Lösungsmittel einen Alkohol enthält, vorzugsweise ausgewählt aus Methanol, Ethanol und Isopropanol, besonders bevorzugt Methanol.

11. Das Verfahren nach einem der vorhergehenden Ansprüche, wobei die Schritte (b) und (c) in demselben Reaktor durchgeführt werden, ohne das 1-Nitroso-2-methyl-2,3-dihydroindol **2** zu isolieren.

12. Das Verfahren nach einem der vorangehenden Ansprüche, wobei das Säureadditionssalz in Schritt (d) das Hydrochlorid ist.

13. Das Verfahren nach einem der vorhergehenden Ansprüche, wobei Schritt (d) das Inkontaktbringen des 1-Amino-2-methyl-2,3-dihydroindols **3** mit HCI in einem Lösungsmittel umfasst; vorzugsweise in Ethylacetat, Ethanol, Isopropanol, Cyclopentylmethylether, Wasser, wässrigem Ethylacetat, wässrigem Ethanol, wässrigem Isopropanol oder wässrigem Cyclopentylmethylether.

14. Ein Verfahren zur Synthese von Indapamid, umfassend das Verfahren zur Synthese von 1-Amino-2-methyl-2,3-dihydroindol **3** oder eines Säureadditionssalzes davon nach einem der vorhergehenden Ansprüche.

15. Das Verfahren nach Anspruch 14, das die folgenden Schritte umfasst
(e) Bereitstellung von 4-Chlor-3-sulphamoylbenzoylchlorid 5 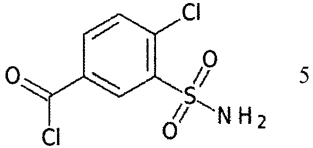 und
(f) Umsetzen des 4-Chlor-3-sulfamoylbenzoylchlorids **5** mit dem 1-Amino-2-methyl-2,3-dihydroindol **3** oder dem Säureadditionssalz davon zur Bildung von Indapamid.

## Revendications

1. Procédé de synthèse du 1-amino-2-méthyl-2,3-dihydroindole **3** ou d'un sel d'addition d'acide de celui-ci, comprenant les étapes suivantes :
(a) fournir du 2-méthyl-2,3-dihydro-1H-indole **1** 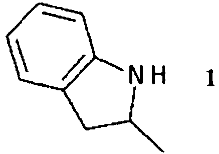
(b) nitrosation du 2-méthyl-2,3-dihydro-1H-indole 1 avec un agent de nitrosation pour obtenir le 1-nitroso-2-méthyl-2,3-dihydroindole 2 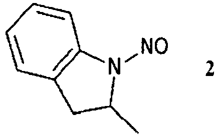
(c) réduire le 1-nitroso-2-méthyl-2,3-dihydroindole 2 avec du dioxyde de thiourée comme agent réducteur pour obtenir le 1-amino-2-méthyl-2,3-dihydroindole 3 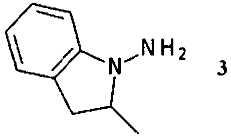 et
(d) éventuellement, convertir le 1-amino-2-méthyl-2,3-dihydroindole **3** en son sel d'addition d'acide.

2. Procédé selon la revendication 1, dans lequel, à l'étape (c), le rapport molaire du dioxyde de thiourée au 1-nitroso-2-méthyl-2,3-dihydroindole **2** est au plus de 8; de préférence au plus de 7; plus préférablement au plus de 6; encore plus préférablement au plus de 5; encore plus préférablement au plus de 4; encore plus préférablement au plus de 3; de préférence au maximum 2,5; et en particulier au maximum 2.

3. Procédé selon la revendication 1 ou 2, dans lequel l'étape (c) est réalisée dans un solvant aqueux.

4. Procédé selon la revendication 3, dans lequel le solvant aqueux contient un alcool, de préférence choisi parmi le méthanol, l'éthanol et l'isopropanol, et plus préférablement le méthanol.

5. Procédé selon la revendication 4, dans lequel le rapport volumique de l'alcool à l'eau est compris dans la plage de 1:5 à 5:1.

6. Procédé selon l'une quelconque des revendications précédentes, dans lequel l'étape (c) est réalisée à une température comprise dans la plage de 40 à 75, de préférence de 45 à 70, plus préférablement de 50 à 70, et de preference 55 à 65.

7. Procédé selon l'une quelconque des revendications précédentes, dans lequel l'étape (c) est réalisée en présence d'une base; de préférence une base inorganique; de préférence un hydroxyde; de préférence un hydroxyde de métal alcalin; de préférence l'hydroxyde de sodium.

8. Procédé selon l'une quelconque des revendications précédentes, dans lequel l'agent nitrosant dans l'étape (b) est un nitrite; de préférence un nitrite de métal alcalin; plus préférablement un nitrite de sodium.

9. Procédé selon l'une quelconque des revendications précédentes, dans lequel l'étape (b) est réalisée dans un solvant aqueux.

10. Procédé selon la revendication 9, dans lequel le solvant aqueux contient un alcool, de préférence choisi parmi le méthanol, l'éthanol et l'isopropanol, et plus préférablement le méthanol.

11. Procédé selon l'une quelconque des revendications précédentes, dans lequel les étapes (b) et (c) sont réalisées dans le même réacteur sans isoler le 1-nitroso-2-méthyl-2,3-dihydroindole **2.**

12. Procédé selon l'une quelconque des revendications précédentes, dans lequel le sel d'addition d'acide dans l'étape (d) est le chlorhydrate.

13. Procédé selon l'une quelconque des revendications précédentes, dans lequel l'étape (d) consiste à mettre en contact le 1-amino-2-méthyl-2,3-dihydroindole **3** avec du HCI dans un solvant; de préférence dans de l'acétate d'éthyle, de l'éthanol, de l'isopropanol, du cyclopentylméthyléther, de l'eau, de l'acétate d'éthyle aqueux, de l'éthanol aqueux, de l'isopropanol aqueux ou du cyclopentylméthyléther aqueux.

14. Procédé de synthèse de l'indapamide comprenant le procédé de synthèse du 1-amino-2-méthyl-2,3-dihydroindole **3** ou d'un sel d'addition d'acide de celui-ci selon l'une quelconque des revendications précédentes.

15. Procédé selon la revendication 14, qui comprend les étapes consistant à
(e) fournir du chlorure de 4-chloro-3-sulfamoylbenzoyle **5** 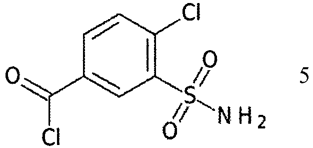 et
(f) faire réagir le chlorure de 4-chloro-3-sulfamoylbenzoyle **5** avec le 1-amino-2-méthyl-2,3-dihydroindole **3** ou son sel d'addition d'acide pour obtenir l'indapamide.
